# EUROPEAN PATENT APPLICATION

(11) **EP 4 516 776 A1**
(43) Date of publication of application: **05.03.2025**
(21) Application number: 22939499.4
(22) Date of filing: 19.05.2022
(51) Int. Cl.: C07D 213/30, C07B 53/00, C07D 401/04, B01J 31/24

(54) **PROCESS FOR UNPROTECTED ASYMMETRIC PREPARATION OF NICOTINE**

(30) Priority: 26.04.2022 CN 202210446666
(71) Applicant: Shenzhen Catalyst Technology Co., Ltd, Shenzhen, Guangdong 518129 (CN); Shenzhen Greencat Pharmaceutical Technology Co., Ltd., Shenzhen, Guangdong 518129 (CN)
(72) Inventor: LANG, Qiwei, Shenzhen, Guangdong 518129 (CN); DING, Xiaobing, Shenzhen, Guangdong 518129 (CN); GAO, Shuang, Shenzhen, Guangdong 518129 (CN)
(74) Representative: Zaboliene, Reda
(86) International application number: PCT/CN2022/093868
(87) International publication number: WO 2023/206665

(57) **Abstract**

The present disclosure relates to an asymmetric process for preparing nicotine without protection, in particular to a process for preparing optically pure nicotine by taking nicotinate as a starting material and carrying out four-step reaction. The process comprises the following steps: nicotinate and γ-butyrolactone are subjected to condensation reaction, asymmetric catalytic reduction reaction, activation, and reaction with methylamine to give optically pure nicotine. The asymmetric catalytic reduction for preparing the chiral alcohol intermediate compound with high optical activity is a key step of the method. The method of the present disclosure has the characteristics of high atom economy, very high reaction activity, capability of keeping excellent stereocontrol, capability of obtaining a chiral product with very high enantioselectivity, short reaction steps, low cost of raw materials, green and pollution-free, capability of greatly reducing the quantity of "three wastes", and easiness in realizing industrial amplification production.

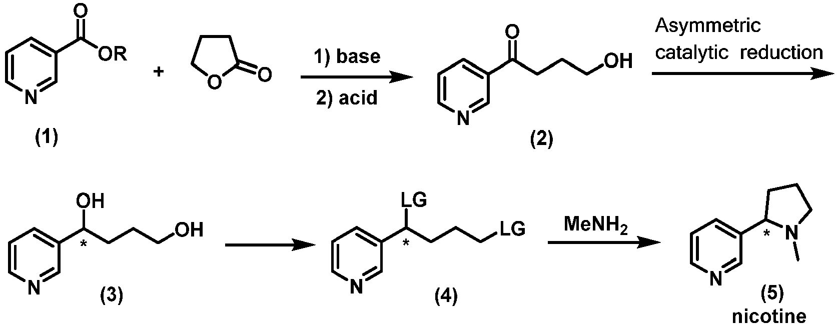

## Description

### Technical field

The present disclosure relates to the technical field of chemical synthesis, and particularly relates to an asymmetric process for preparing nicotine without protection.

### Background

Nicotine is widely found in tobacco plants and various Solanaceae plants and has unique biological activity. In agricultural production, nicotine is widely used as a pesticide. In the field of medicine, clinical researches have shown that nicotine can act on acetylcholine receptors and is expected to become an effective drug for senile dementia, Parkinson's disease, schizophrenia, depression, and other central nervous system diseases. Nicotine can also be used as a drug to reduce health problems caused by smoking. Medical staff give a measured amount of nicotine to smokers, and the nicotine may be chewing type, patch, troche, cigarette substitute, nasal spray, or the like. In addition, in the field of chemical synthesis, researches report that nicotine can also be used as a chiral ionic liquid to participate in various asymmetric chemical reactions.

The natural nicotine, i.e., L-nicotine, has 10-100 times the affinity for acetylcholine receptors of D-nicotine, and is more widely used. However, nicotine used in the current market is derived primarily from plant extraction. The source of nicotine is influenced by various factors such as raw material, climate, soil, and period, and it is not possible to meet the market demand for nicotine solely by extracting it from plants. Therefore, the realization of large-scale production of nicotine by means of chemical synthesis preparation technology is of great significance. The chemical structure of nicotine is shown as follows:

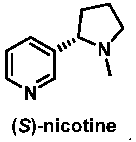

Research on the chemical synthesis of nicotine has long been the focus of attention of scientists. Natural nicotine was first isolated from tobacco in 1828 by the German chemists Posselt and Reimann and was first obtained in a laboratory in 1904 by A.Pictet and Crepieux in a synthetic manner. After the development of more than one hundred years, a plurality of research reports of chemical preparation of nicotine appear. The existing chemical synthesis methods of nicotine are mainly divided into two types:

The first type is to synthesize racemic nicotine first and then obtain optical pure nicotine by a chiral resolution method. This method has simple synthesis steps, but needs a large amount of chiral resolving reagents, so that the separation and purification operations become complicated and the cost is relatively high. See documents 1) Journal of Organic Chemistry, 1990, 55, 1736-1744; 2) Journal of the Chemical Society, Perkin Transactions I, 2002 (2), 143-154; and 3) Synlett, 2009 (15), 2497-2499; document Journal of Heterocyclic Chemistry, 2009, 46 (6),1252-1258; patents 1) CN 102617547; 2) CN 107406411; 3) CN 110256403; and the like.

The second type is to directly obtain nicotine by an asymmetric synthesis without additional chiral resolving reagents, which can directly obtain optically active nicotine, but these methods are very expensive for large-scale production of nicotine and no commercial synthetic route has emerged.

For example, Chavdarian et al. reported asymmetric synthesis of nicotine (Journal of Organic Chemisry, 1982, 47, 1069-1073) for the first time. They prepared a module of chiral aminoalcohol using L-Proline as the starting material and then obtained the target product (S)-nicotine through five steps of reaction. However, the *ee* value is only 24% (reaction formula 1).

### Reaction formula 1:

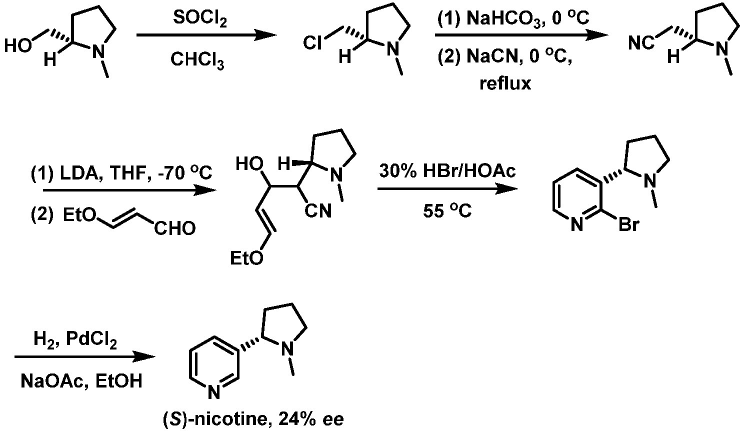

Helmchen et al. accomplished asymmetric synthesis of (S)-nicotine by a strategy of iridium-catalyzed allylic reductive amination (Organic & Biomolecular Chemistry, 2005, 3, 3266-3268), and the *ee* value is up to 99% (reaction formula 2). Its drawback is the use of precious metals iridium and ruthenium, with large quantities required, leading to high costs and making industrialization difficult.

### Reaction formula 2:

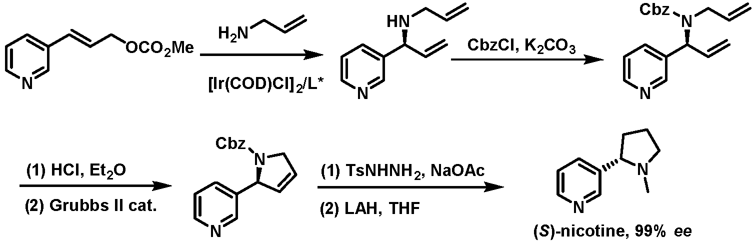

O'Brien et al. completed the asymmetric synthesis of (S)-nicotine starting from the simple and readily available starting material N-Boc-tetrahydropyrrole by selective lithiation under the action of chiral diamine, transmetalation, and palladium-catalyzed Negishi coupling reaction (Journal of Organic Chemistry, 2011, 76 (15), 5936-5953), and the *ee* value is up to 84% (reaction formula 3).

### Reaction formula 3:

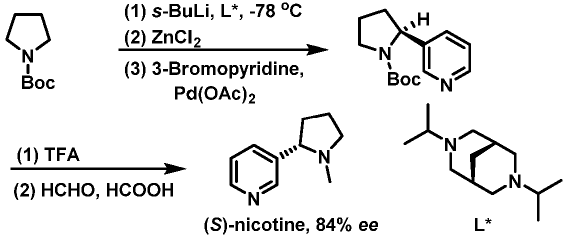

M. Ortiz-Marciales et al. reported that nicotine and anabasine can be synthesized from 3-bromopyridine as a starting material by 4-step reaction (Journal of Heterocyclic Chemistry, 2009, 46, 1252-1258.). The method has the advantage of simple steps in the synthesis of racemic nicotine and anabasine (reaction formula 4). However, when the method is applied to the synthesis of optically pure alkaloid of this type, not only the expensive CBS reducing agent and TIPSCl protecting agent are used, but also additional protecting and deprotecting operations are added, which results in increased steps and costs. In addition, when the corresponding alkaloid is formed in ring closing, the optical purity of the product is greatly reduced, that is, the initial 94% ee is reduced to 82% ee, so that the application value of the method is reduced (reaction formula 5).

### Reaction formula 4:

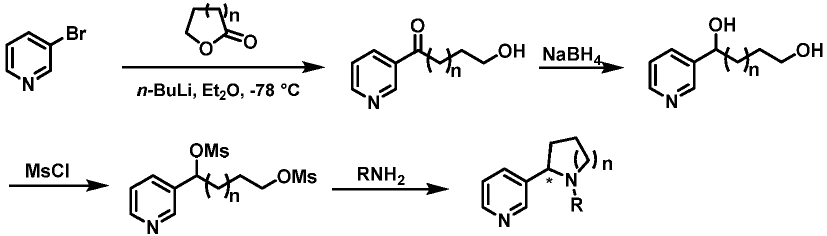

### Reaction formula 5:

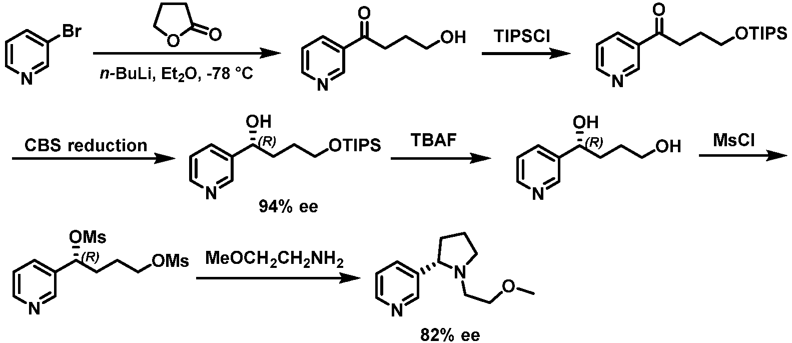

In combination with the above documents and patents, the existing asymmetric synthesis methods of nicotine have many disadvantages. For example, the expensive CBS reducing agent is used, or a n-butyllithium reagent which is relatively harsh in operation is required, and the low-temperature reaction condition is used, which increases energy consumption. In addition, some methods require protection and deprotection operations, which are not only expensive in terms of the protecting reagent used, but also increase the number of steps, resulting in complicated separation and purification operations and increased production costs and equipment costs.

Therefore, in order to be beneficial to the industrial synthesis of optical pure nicotine, the method for synthesizing nicotine by artificial asymmetry is further improved. The production costs and the discharge of "three wastes" are reduced, and the product quality is improved, so that the method is of important significance.

### Summary

Aiming at the technical problems of expensive reaction reagents, harsh reaction conditions, complicated reaction steps, poor chiral selectivity, difficult separation and purification, and the like in the method described above for preparing optically pure nicotine, the present disclosure provides an asymmetric method for preparing nicotine without protection, which has the characteristics of high atom economy, very high reaction activity, capability of keeping excellent stereocontrol, capability of obtaining a chiral product with very high enantioselectivity, short reaction steps, low cost of raw materials, green and pollution-free, capability of greatly reducing the quantity of "three wastes", and easiness in realizing industrial amplification production.

The present disclosure provides a method for preparing nicotine. Nicotine, known as compound (5), has the structure shown below:

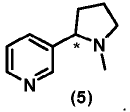

In the preparation method provided by the present disclosure, compound (1) can be used as a starting material to perform condensation reaction with γ-butyrolactone to give compound (2); hydrogen compound (2) is subjected to asymmetric catalytic reduction reaction to give compound (3); compound (3) is activated to give compound (4); compound (4) is reacted with methylamine to give compound (5), namely nicotine. The specific reaction route is as follows:

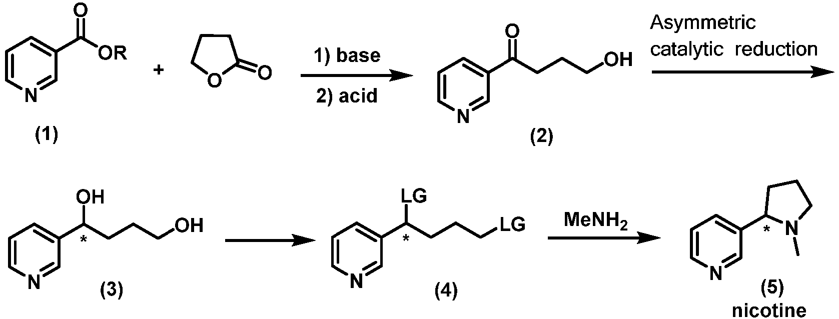

The present disclosure provides an asymmetric catalytic synthesis method for nicotine, which is realized by the following technical scheme:

In one aspect, the present disclosure provides a method for preparing a compound of formula (3): 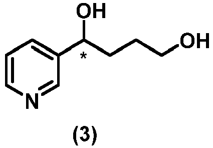
the compound has an R or S configuration at the stereoisomer center labeled with *;
an enantiomer excess of at least 70% relative to the opposite enantiomer,
wherein the method comprises the following steps: asymmetrically reducing the intermediate compound represented by formula (2):

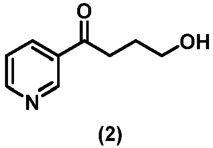

The asymmetric reduction is carried out in a suitable organic solvent in the presence of a chiral metal catalyst, a chiral ligand, a transition metal, an additive, and a hydrogen source. The hydrogen source is selected from at least one of hydrogen, formic acid, a mixture of formic acid and formate, and a mixture of formic acid and organic amine, and the transition metal is selected from at least one of ruthenium, rhodium, iridium, palladium, manganese, copper, and iron.

In the present disclosure, the asymmetric reduction reaction may be achieved by the following three schemes:

Scheme 1: The asymmetric catalytic reduction method may be a ruthenium-diphosphine-diamine catalytic system. Under the condition, compound (2) is in a reaction solvent, a catalyst and a base are added, and the asymmetric hydrogenation reaction is carried out under high-pressure hydrogen to give compound (3). The general structural formula of the catalyst is: 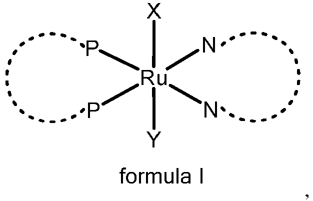
X and Y are each independent halogen or acetate or hydrogen; 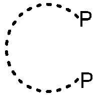 refers to a diphosphine ligand, and 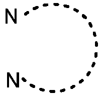 refers to a diamine structure;
specifically, the diamine structure is selected from any one of the following structures or corresponding isomers thereof:

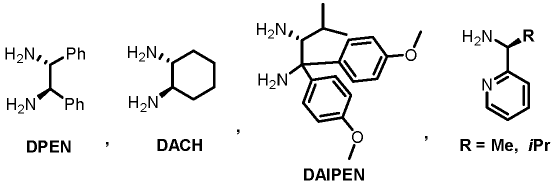

The diphosphine ligand is selected from Binap, H₈-Binap, MeO-Biphep, C₃*-Tunephos, Segphos, Synphos, SunPhos, Difluophos, P-Phos, BPE, DIPAMP, DIOP, Duphos, SDP, and O-SDP, and corresponding isomers thereof or derivatives thereof, and specifically, the structure of the diphosphine ligand is represented as:

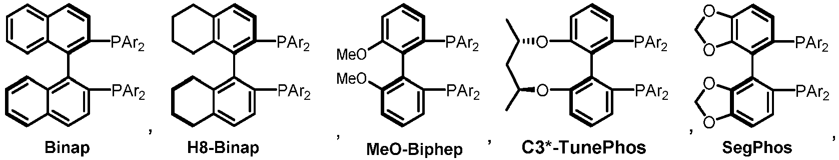

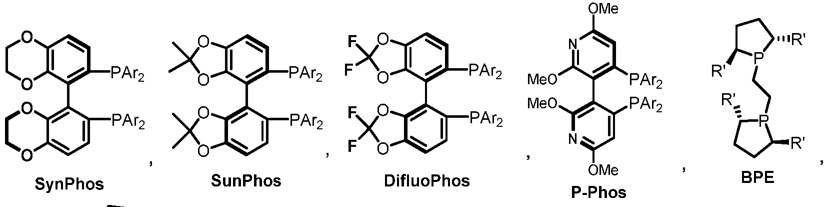

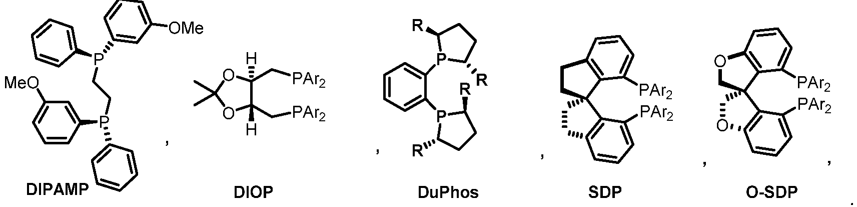

According to the ruthenium-diphosphine-diamine catalyst described above, the structure is represented as follows:

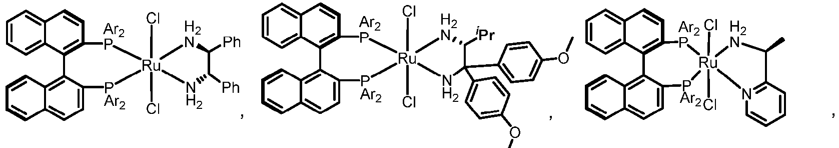

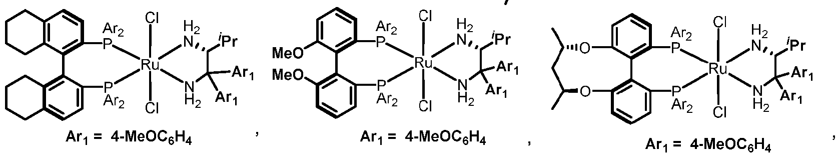

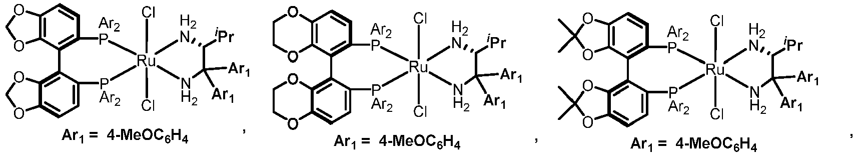

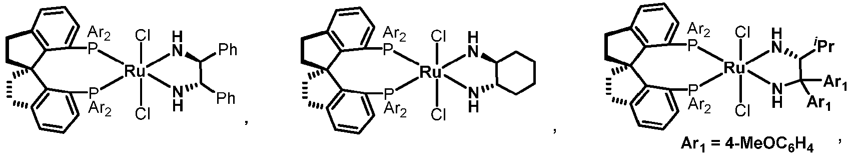

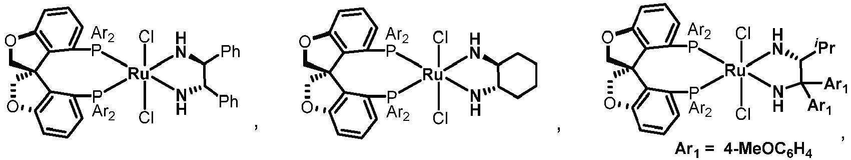

The Ar group in the diphosphine ligand represents aryl, namely phenyl, 4-methylphenyl, 3,5-dimethylphenyl, 3,5-di-tert-butylphenyl, or 3,5-di-tert-butyl 4-methoxyphenyl.

Scheme 2: In the present disclosure, the asymmetric catalytic reduction reaction can also be realized by a polydentate ligand catalytic system, and the specific scheme is that: compound (2) is in a suitable reaction solvent, a catalyst and a base are added, and the asymmetric hydrogenation reaction is carried out under high-pressure hydrogen to give compound (3).

The catalyst may also be obtained by *in-situ* complexing of a metal compound and a chiral polydentate ligand. The metal salt of the catalyst is selected from common transition metal compounds such as ruthenium, rhodium, iridium, and palladium. The chiral polydentate ligand is selected from L1-L27:

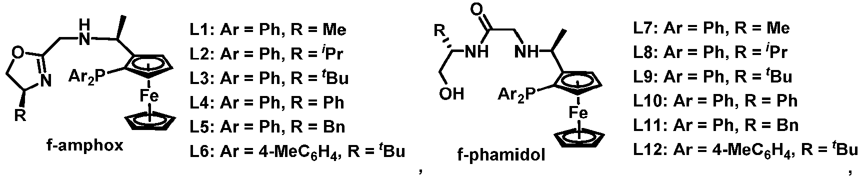

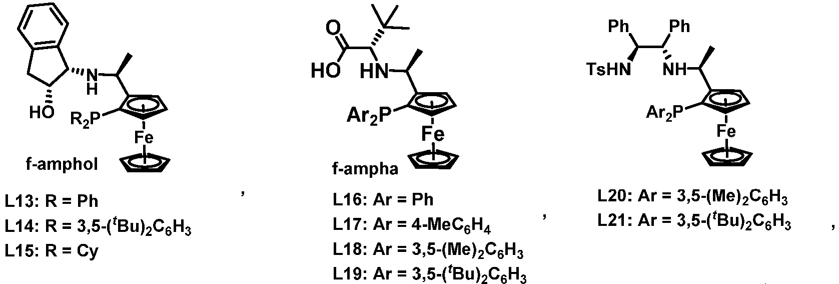

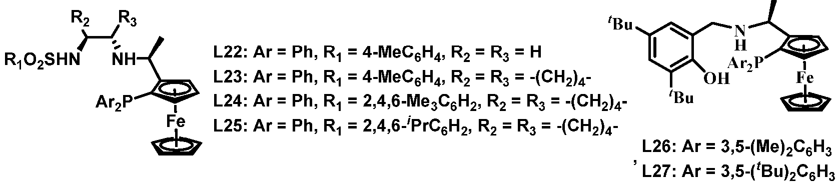

As a preferred embodiment of the present disclosure, the transition metal catalyst is preferably [Ir(COD)Cl]₂ complexed with a chiral ligand, and the chiral ligand is preferably L9:

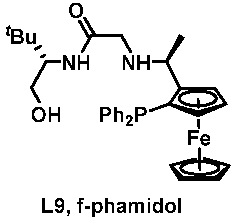

As a preferred condition of the first and second schemes of the present disclosure, the hydrogen source in the asymmetric catalytic reduction reaction described above is hydrogen; the solvent used is at least one of methanol, ethanol, isopropanol, tetrahydrofuran, dichloromethane, and toluene, preferably an alcohol solvent; the additive used is a base selected from at least one of potassium tert-butoxide, sodium tert-butoxide, lithium *tert-*butoxide, potassium hydroxide, sodium hydroxide, sodium carbonate, potassium carbonate, and cesium carbonate, and the base is preferably a potassium salt.

As a preferred condition of the first and second schemes of the present disclosure, the temperature of the reaction is 20-80 °C, the hydrogen pressure is 1-8 Mpa, and the reaction time is 8-60 h. The molar ratio of intermediate (2) to the catalyst is 2000:1 to 200000:1, more preferably 10000:1 to 100000:1.

Scheme 3: In the present disclosure, the asymmetric catalytic reduction method can also be realized by an asymmetric transfer hydrogenation catalytic method, that is, compound (2) is in a reaction solvent, a mixture of a catalyst and a hydrogen source is added to carry out the asymmetric transfer hydrogenation reaction to give compound (3), and the structure of the catalyst used in the transfer hydrogenation process is as follows:

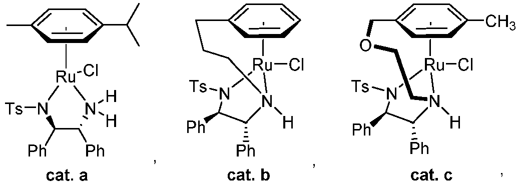

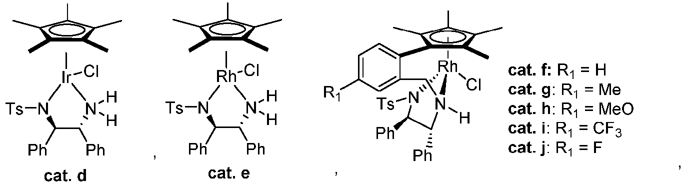

As a preferred condition of the third scheme of the present disclosure, the hydrogen source of the catalyst used in the transfer hydrogenation process is selected from HCOOH/Et₃N, HCOOH/DIPEA, HCOOH/*ⁱ*Pr₂NH, HCOOH/Et₂NH, HCOOH/DBU, HCOOH/HCOOK, and HCOOH/HCOONa; more preferably, the hydrogen source is HCOOH/Et₃N, HCOOH/DIPEA, HCOOH/*ⁱ*Pr₂NH, HCOOH/Et₂NH, HCOOH/HCOOK, or HCOOH/HCOONa at a molar ratio of 5:2. The solvent for the catalyst used in the transfer hydrogenation process is selected from EtOAc, CH₂Cl₂, ClCH₂CH₂Cl, MeOH, EtOH, *ⁱ*PrOH, (HOCH₂)₂, THF, and PhMe, and the solvent is preferably an alcohol solvent, more preferably EtOH, or EtOH/H₂O or MeOH at a volume ratio of 1:1.

The present disclosure further provides an intermediate compound for preparing chiral nicotine, and the structure of the compound is as shown in the following formula (3): 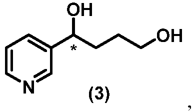 and specifically comprises two configurations (R) and (S), wherein the structures thereof are shown below,

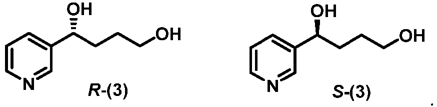

As a preferred technical scheme of the present disclosure, optically pure compounds R-(3) and S-(3) are preferred, more preferably compound R-(3).

The present disclosure further provides an asymmetric process for preparing nicotine without protection, and the synthetic route is as follows:

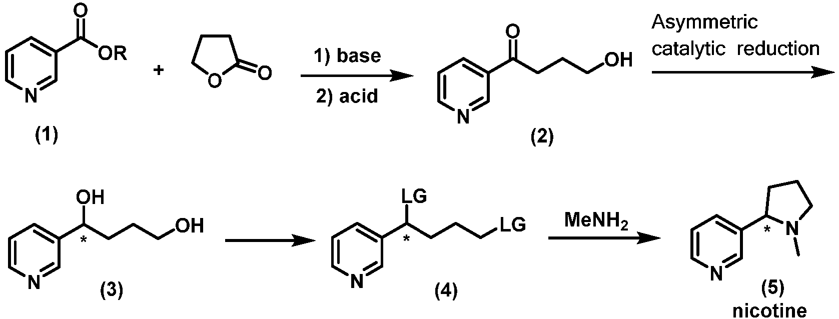

Intermediate (3) is prepared by the synthesis method of any scheme of the above asymmetric reduction methods.

Specifically, the following steps are included: 1) heating nicotinate (1) and γ-butyrolactone to 70-120 °C in a proper solvent and performing a condensation reaction under the action of a base, and performing a ring-opening decarboxylation reaction on the condensation intermediate under the action of an acid to give a hydrogenation precursor compound (2); 2) dissolving intermediate (2) in a proper solvent, adding a chiral catalyst and a proper additive, selecting a proper hydrogen source, and performing an asymmetric reduction reaction to give a chiral diol product (3); 3) activating the chiral diol product (3) to form a compound (4), wherein LG represents a suitable leaving group; 4) under suitable conditions, reacting intermediate (4) with methylamine to form optically pure nicotine (5).

As a preferred technical scheme of the present disclosure, R in the nicotinate formula (1) represents alkyl, more preferably methyl and ethyl ester.

As a preferred technical scheme of the present disclosure, the base used in the condensation reaction of the nicotinate formula (1) and γ-butyrolactone is selected from at least one of sodium methoxide, sodium ethoxide, sodium tert-butoxide, potassium tert-butoxide, and sodium hydride.

As a preferred technical scheme of the present disclosure, the solvent used in the condensation reaction is selected from tetrahydrofuran, 2-methyltetrahydrofuran, diethylene glycol dimethyl ether, ethylene glycol dimethyl ether, toluene, xylene, and benzene, and an ether solvent is preferred, i.e., tetrahydrofuran, 2-methyltetrahydrofuran, diethylene glycol dimethyl ether, and ethylene glycol dimethyl ether, more preferably tetrahydrofuran and 2-methyltetrahydrofuran.

As a preferred technical scheme of the present disclosure, the condensation intermediate is subjected to a ring-opening decarboxylation reaction under the action of an acid. The acid is selected from one or a mixture of hydrochloric acid, sulfuric acid, phosphoric acid, acetic acid, and trifluoroacetic acid in any proportion, preferably hydrochloric acid, sulfuric acid, and phosphoric acid, and more preferably hydrochloric acid and sulfuric acid.

As a preferred technical scheme of the present disclosure, the leaving group LG in compound (4) represents halogen, sulfonate, and the like, wherein the sulfonate includes methanesulfonate (OMs), trifluoromethanesulfonate (OTf), p-toluenesulfonate (OTs), nitrosulfonate (ONs), and the like, preferably methanesulfonate (OMs) and p-toluenesulfonate (OTs). LG is preferably chlorine, methanesulfonate (OMs), and p-toluenesulfonate (OTs).

As a preferred technical scheme of the present disclosure, intermediate (4) is reacted with methylamine to form optically pure nicotine (5), and the methylamine is selected from aqueous, alcohol, and ether solutions of methylamine, preferably an alcohol solution of methylamine; the reaction temperature is -20 °C to 10 °C, more preferably -10 °C to 5 °C.

Compared with the prior art, the present disclosure has the following beneficial effects:
(1) The present disclosure successfully develops an asymmetric process for preparing nicotine without protection. The process has no protective group, short steps, simple and convenient operation, low cost, and less "three wastes" generated, conforms to the concept and requirement of green chemistry, is easy to amplify, and is suitable for industrial production.
(2) In the process, the asymmetric catalytic reduction reaction is a key step, which can be realized by three schemes disclosed by us, so that a chiral alcohol intermediate is effectively constructed; the reaction has good stability and reaction activity, the proportion and quantity of the catalyst are low, excellent stereocontrol is realized, and the chiral alcohol intermediate with the enantioselectivity of more than 99% can be obtained.
(3) It was found by research that by using the most preferred catalyst system Ir/f-phamidol, the asymmetric hydrogenation reaction has a very high reaction activity, the catalyst conversion number (TON, turnover number) is up to 200000, and excellent stereocontrol can be maintained.
(4) By the process for preparing nicotine disclosed by the patent, nicotine with different configurations can be synthesized by controlling the chirality of the ligand in the asymmetric reduction step, which has a flexible formulating function. The nicotine prepared by the process has high optical purity (99% ee), excellent product quality (> 99.5% chemical purity), and relatively good application value.

### Brief description of the drawings

FIG. 1 is a schematic diagram of a nicotine asymmetric synthesis process.
FIG. 2 is a ¹H NMR spectrum of compound (2).
FIG. 3 is a ¹³C NMR spectrum of compound (2).
FIG. 4 is a ¹H NMR spectrum of compound (3).
FIG. 5 is a ¹³C NMR spectrum of compound (3).
FIG. 6 is a ¹H NMR spectrum of compound (5).
FIG. 7 is a ¹³C NMR spectrum of compound (5).
FIG. 8 is an HPLC chromatogram of racemic compound (3).
FIG. 9 is an HPLC chromatogram of chiral compound (3).
FIG. 10 is an HPLC chromatogram of racemic compound (5).
FIG. 11 is an HPLC chromatogram of chiral compound (5).

### Detailed description

The present disclosure will be further described with reference to the specific examples and figures, but the present disclosure is not limited thereto.

The experimental methods in the examples, in which specific conditions are not specified, are generally performed under the conventional conditions and the conditions described in the manual or under the conditions recommended by the manufacturer; the materials, reagents, and the like used are commercially available unless otherwise specified.

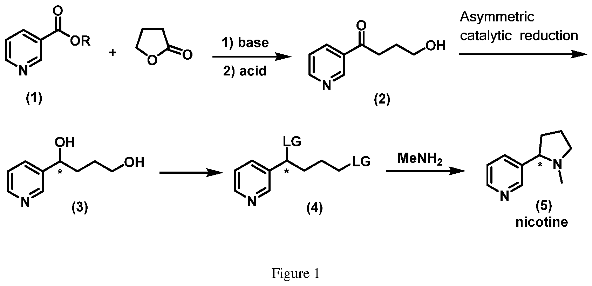

The asymmetric synthesis process schematic diagram referring to FIG. 1:

### Example 1 Synthesis of Intermediate (2) (reference: Journal of Heterocyclic Chemistry, 2006, 49, 1252-1258.)

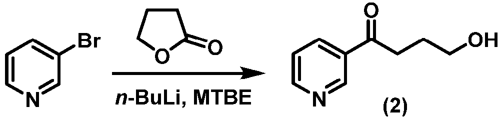

3-Bromopyridine (15.8 g, 100 mmol) was added to a three-necked round-bottom flask under argon atmosphere, dissolved with 250 mL of methyl *tert-*butyl ether (MTBE), and stirred and cooled to -78 °C in a low-temperature tank. 48 mL of a solution of n-butyl lithium (2.4 M, 120 mmol) in n-hexane was then slowly added dropwise, and the temperature was maintained at -78 °C during the dropwise addition. After the dropwise addition, the temperature was maintained at -78 °C, and the mixture was stirred for 30 min. γ-Butyrolactone (8.46 mL, 110 mmol) was dissolved with 50 mL of methyl *tert-*butyl ether (MTBE) and then added dropwise to the reaction mixture. The temperature was maintained at -78 °C, and the mixture was stirred for 2 h, slowly warmed to room temperature, and reacted for 1 h. The reaction was quenched with 50 mL of dilute hydrochloric acid (2 M), and the mixture was extracted with ethyl acetate. The organic phase was washed with saturated sodium bicarbonate and saturated brine, dried over anhydrous sodium sulfate, and concentrated to dryness by rotary evaporation to give a crude product, which was separated by column chromatography to give pure intermediate (2), 69% yield.

¹H NMR (600 MHz, Chloroform-*d*) δ 9.18 (s, 1H), 8.80 - 8.72 (m, 1H), 8.25 (d, *J* = 7.7 Hz, 1H), 7.47 - 7.41 (m, 1H), 3.76 (t, *J* = 5.7 Hz, 2H), 3.16 (t, *J* = 6.9 Hz, 2H), 2.78 (s, 1H), 2.04 (q, *J* = 6.2 Hz, 2H). ¹³C NMR (151 MHz, CDCl₃) δ 199.11, 153.25, 149.43, 135.41, 132.08, 123.63, 61.61, 35.38, 26.56.

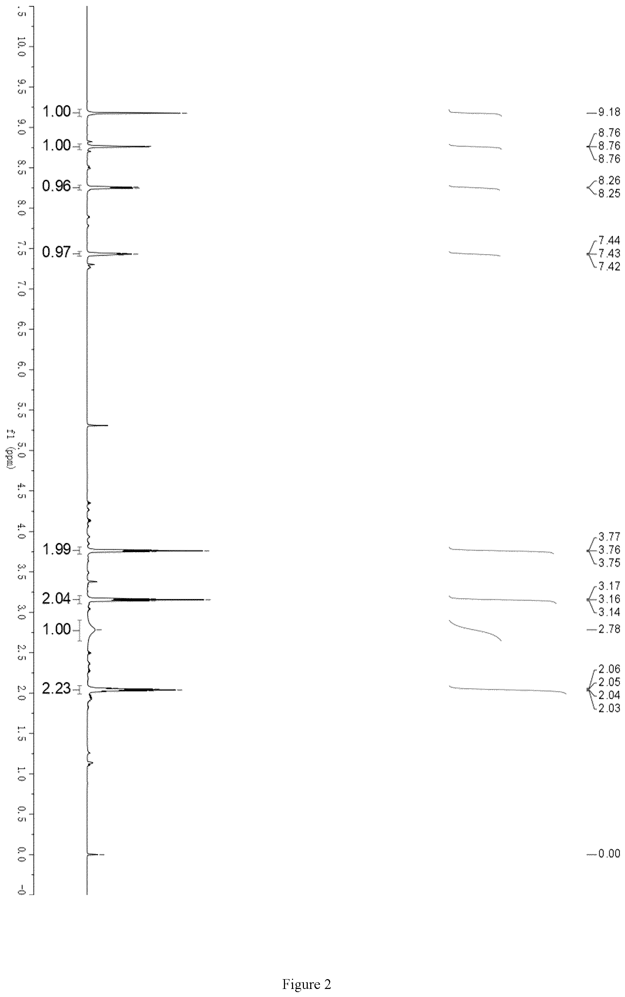

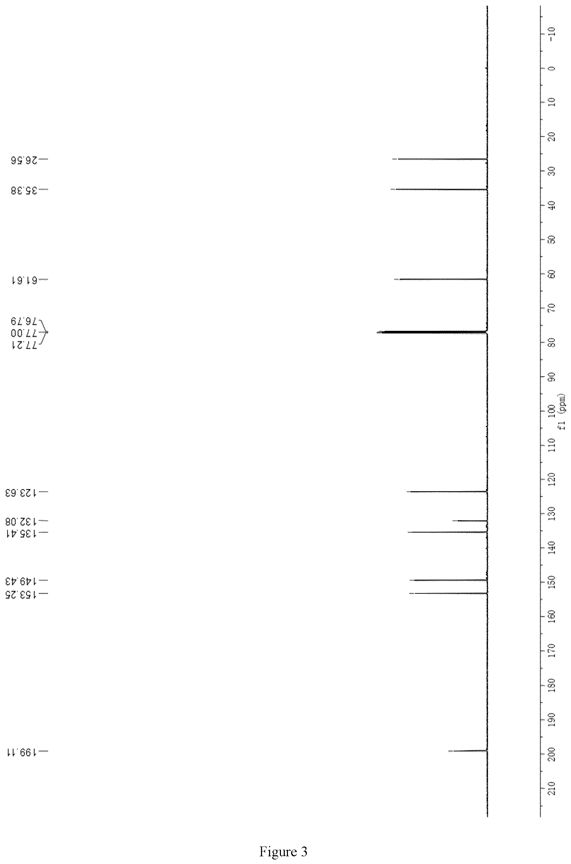

FIG. 2 is a ¹H NMR spectrum of compound (2), and FIG. 3 is a ¹³C NMR spectrum of compound (2).

### Example 2 Synthesis of Intermediate (2)

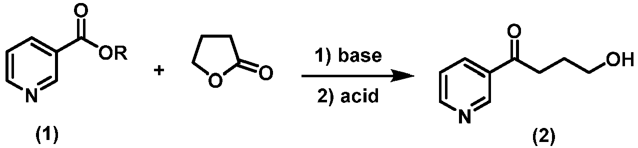

### Example 2-1 Condensation Reaction:

A base (198.5 mmol) and nicotinate (132.3 mmol) were dissolved in 200 mL of a corresponding solvent at room temperature, and γ-butyrolactone (185.2 mmol) was diluted with 50 mL of the reaction solvent and added dropwise to the reaction system. After the addition was completed, the mixture was reacted at 75-100 °C overnight. After the reaction was completed, the mixture was returned to the room temperature and quenched with water. The pH of the system was neutralized to 7±1. The system was precipitated. The aqueous phase was extracted three times with DCM, dried over anhydrous sodium sulfate, filtered and concentrated to dryness by rotary evaporation to give a condensation product, which was directly used in the next reaction. In this reaction, the effect of the starting materials nicotinate, base, and reaction solvent was investigated. The results are shown in Table 1 below.

**Table 1**

| No. | Nicotinate | Base | Reaction solvent | Yield (%) |
|---|---|---|---|---|
| 1 | Ethyl nicotinate | Sodium *tert*-butoxide | Tetrahydrofuran | 88 |
| 2 | Ethyl nicotinate | Potassium *tert-*butoxide | Tetrahydrofuran | 86 |
| 3 | Ethyl nicotinate | Sodium methoxide | Tetrahydrofuran | 74 |
| 4 | Ethyl nicotinate | Sodium ethoxide | Tetrahydrofuran | 78 |
| 5 | Ethyl nicotinate | Sodium hydride | Tetrahydrofuran | 82 |
| 6 | Ethyl nicotinate | Sodium *tert*-butoxide | 2-Methyltetrahydrofuran | 89 |
| 7 | Ethyl nicotinate | Sodium *tert*-butoxide | Diethylene glycol dimethyl ether | 90 |
| 8 | Ethyl nicotinate | Sodium *tert*-butoxide | Ethylene glycol dimethyl ether | 85 |
| 9 | Ethyl nicotinate | Sodium *tert*-butoxide | Toluene | 85 |
| 10 | Ethyl nicotinate | Sodium *tert*-butoxide | Xylene | 82 |
| 11 | Methyl nicotinate | Sodium *tert*-butoxide | Tetrahydrofuran | 84 |

### Example 2-2 Hydrolytic Decarboxylation Reaction:

The condensation product (8.12 g, 42.5 mmol) prepared in Example 2-1 described above was added to a diluted acid solution, and the mixture was refluxed at 100-110 °C for 24 h. After the reaction was completed, the system was cooled to 0 °C, and the pH of the system was adjusted to greater than 11 with 6 M sodium hydroxide solution. The system was extracted three times with DCM (3 × 100 mL), dried over anhydrous sodium sulfate, filtered and concentrated to dryness by rotary evaporation to give intermediate (2). In this reaction, the effect of different acids on this reaction was investigated. The results are shown in Table 2 below.

**Table 2**

| No. | Acid + water | Yield (%) |
|---|---|---|
| 1 | HCl (36%, 20 mL) + H₂O (20 mL) | 60 |
| 2 | H₂SO₄ (98%, 5 mL) + H₂O (30 mL) | 78 |
| 3 | H₃PO₄ (85%, 6.4 mL) + H₂O (30 mL) | 75 |
| 4 | HOAc (30 mL) + H₂O (0 mL) | 52 |
| 5 | H₂SO₄ (98%, 5 mL) + HOAc (10 mL) + H₂O (20 mL) | 86 |

### Example 3 Preparation of Chiral Alcohol Intermediate Compound (3) (Ruthenium-Diphosphine-Diamine Catalytic System Investigation)

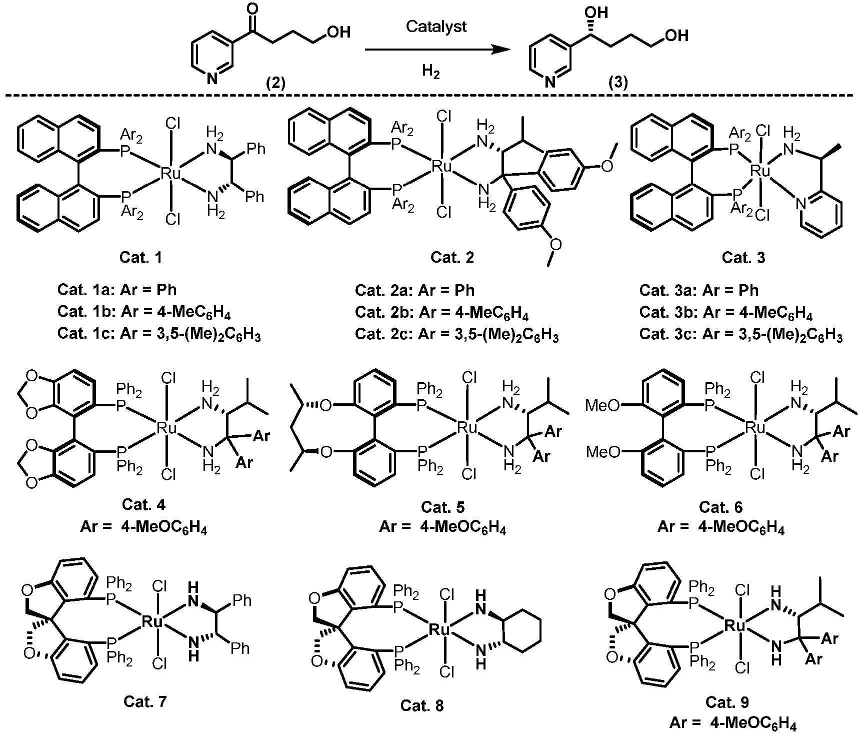

0.63 g of intermediate 2 (3.8 mmol), 2 mL of isopropanol, and 4.3 mg of potassium tert-butoxide (0.038 mmol) were added to a 50 mL reaction kettle under argon atmosphere, and 0.0002 mmol of the catalyst **Cat.** was finally added. The gas in the autoclave was purged with hydrogen three times, and finally 50 atm of hydrogen was introduced, and the mixture was reacted at 25 °C for 16 h. After the reaction was completed, the gas in the autoclave was slowly released, and the mixture was concentrated under reduced pressure to give 0.63 g of a yellow oily liquid, which was hydrogenation product (3).

*R*-(3) characterization data: [α]²⁵_{D} = +45.8 (c = 1.0, CHCl₃); ¹H NMR (600 MHz, Chloroform-d) δ 8.42 (s, 1H), 8.34 - 8.28 (m, 1H), 7.69 (d, *J* = 7.8 Hz, 1H), 7.26 - 7.17 (m, 1H), 4.89 (br, 2H), 4.69 (t, *J =* 5.9 Hz, 1H), 3.60 (ddt, *J =* 18.4, 13.8, 7.0 Hz, 2H), 1.80 (q, *J =* 6.3 Hz, 2H), 1.62 (ddq, J = 26.8, 13.3, 6.6 Hz, 2H). ¹³C NMR (151 MHz, CDCl₃) δ 147.70, 147.08, 140.85, 134.08, 123.52, 71.24, 61.97, 36.23, 28.81.

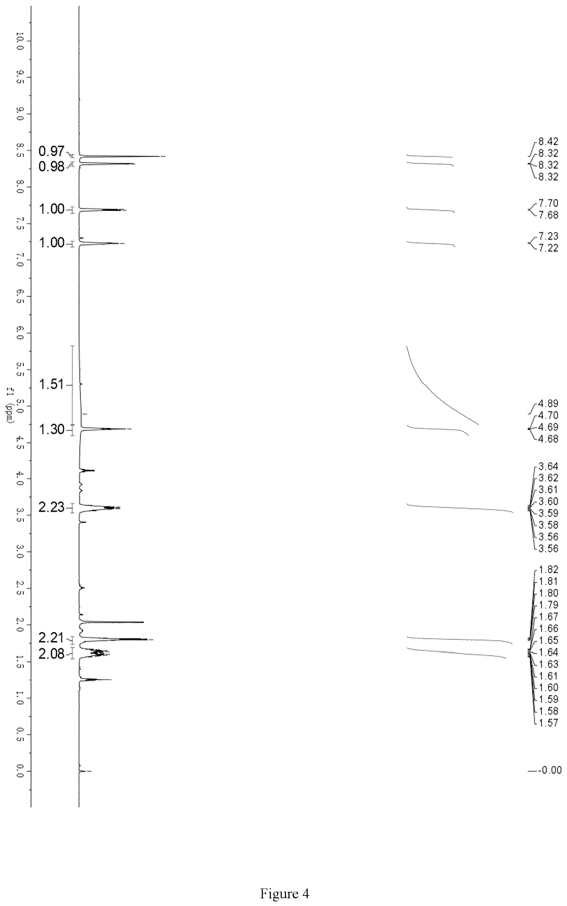

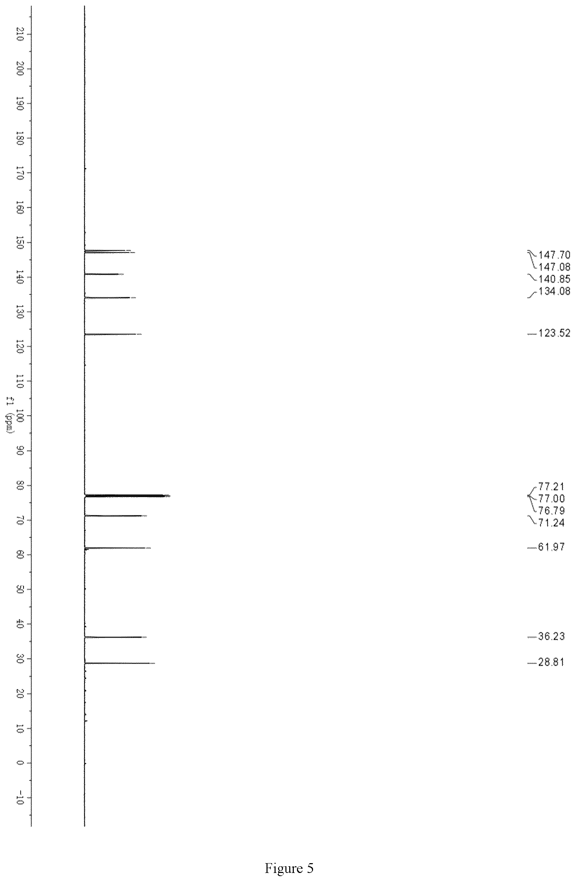

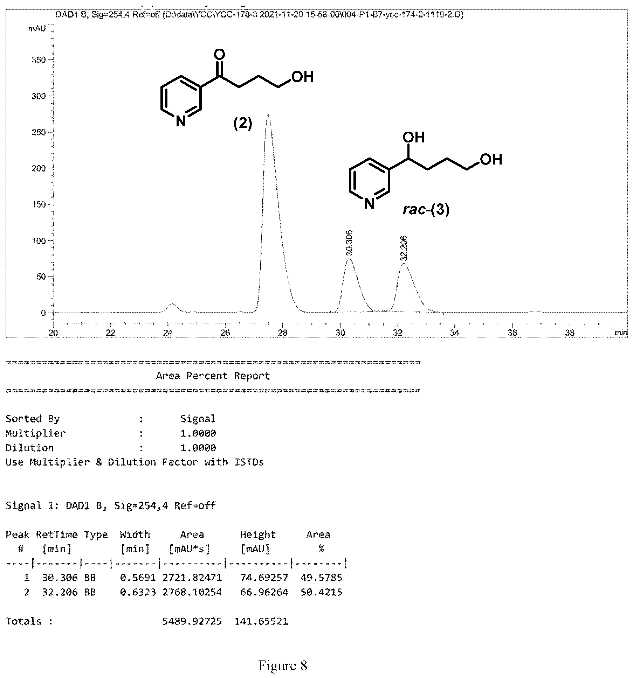

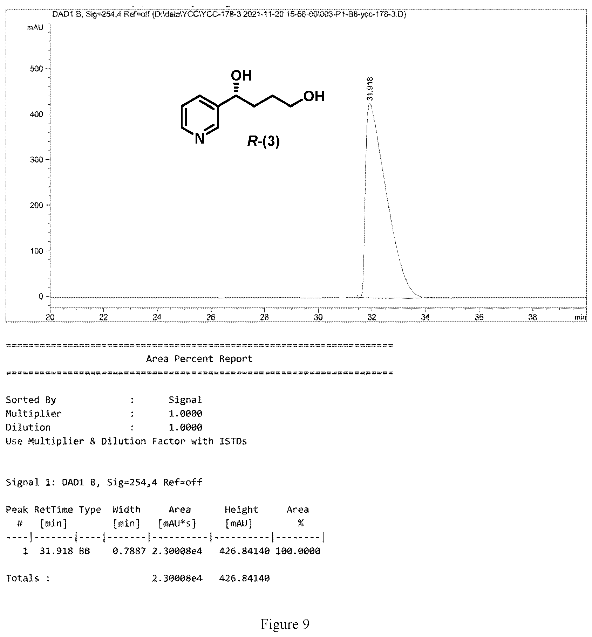

FIG. 4 is a ¹H NMR spectrum of compound (3), and FIG. 5 is a ¹³C NMR spectrum of compound (3). FIG. 8 is an HPLC chromatogram of racemic compound (3); FIG. 9 is an HPLC chromatogram of chiral compound (3); the conversion rate and ee value were determined by HPLC, and the results are shown in Table 3 below.

**Table 3**

| No. | Catalyst (S/C = 5000) | Conversion rate (%) | ee (%) |
|---|---|---|---|
| 1 | **Cat. 1a** | >99 | 57 |
| 2 | **Cat. 1b** | >99 | 65 |
| 3 | **Cat. 1c** | >99 | 80 |
| 4 | **Cat. 2a** | >99 | 93 |
| 5 | **Cat. 2b** | >99 | 97 |
| 6 | **Cat. 2c** | >99 | 99 |
| 7 | **Cat. 3a** | >99 | 34 |
| 8 | **Cat. 3b** | >99 | 45 |
| 9 | **Cat. 3c** | >99 | 50 |
| 10 | **Cat. 4** | >99 | 95 |
| 11 | **Cat. 5** | >99 | 97 |
| 12 | **Cat. 6** | >99 | 98 |
| 13 | **Cat. 7** | >99 | 92 |
| 14 | **Cat. 8** | >99 | 84 |
| 15 | **Cat. 9** | >99 | 96 |

Example 4 To further investigate the effect of the solvent in the reaction system on the reaction, the asymmetric hydrogenation reaction was catalyzed by **Cat. 2c** on the basis of Example 2, and the solvent *ⁱ*PrOH was sequentially replaced by MeOH, EtOH, DCM, THF, hexane, toluene, etc. The reaction time was 16 h, S/C = 5000. The results of the effect of different solvents on the conversion rate and ee value of the reduction of compound (2) are shown in Table 4 below. The conversion rate (conv.) and the enantioselectivity (ee) were determined by HPLC.

**Table 4**

| No. | Catalyst (n mol%) | Reaction solvent | Conversion rate (%) | ee (%) |
|---|---|---|---|---|
| 1 | **Cat. 2c** (0.02 mol %) | *ⁱ*PrOH | >99 | 99 |
| 2 | **Cat. 2c** (0.02 mol %) | MeOH | >99 | 98 |
| 3 | **Cat. 2c** (0.02 mol %) | EtOH | >99 | 99 |
| 4 | **Cat. 2c** (0.02 mol %) | DCM | 97 | 96 |
| 5 | **Cat. 2c** (0.02 mol %) | THF | 91 | 98 |
| 6 | **Cat. 2c** (0.02 mol %) | Hexane | 80 | 96 |
| 7 | **Cat. 2c** (0.02 mol %) | Toluene | 95 | 98 |

Example 5 To investigate the effect of the base added to the reaction system on the reaction, the asymmetric hydrogenation reaction was similarly catalyzed by **Cat. 2c** on the basis of Example 2 and Example 3 using isopropanol as the solvent, and potassium *tert*-butoxide was sequentially replaced by potassium carbonate, cesium carbonate, potassium hydroxide, sodium hydroxide, sodium methoxide, potassium methoxide, sodium *tert-*butoxide, and lithium *tert*-butoxide.The reaction time was 16 h, S/C = 5000. The following experiment was performed. The results are shown in Table 5 below.

**Table 5**

| No. | Base | Reaction solvent | Conversion rate (%) | ee (%) |
|---|---|---|---|---|
| 1 | *^{t}*BuOK | *ⁱ*PrOH | >99 | 99 |
| 2 | K₂CO₃ | *ⁱ*PrOH | 23 | - |
| 3 | Cs₂CO₃ | *ⁱ*PrOH | 30 | - |
| 4 | KOH | *ⁱ*PrOH | 50 | - |
| 5 | NaOH | *ⁱ*PrOH | 41 | - |
| 6 | NaOMe | *ⁱ*PrOH | 67 | 98 |
| 7 | KOMe | *ⁱ*PrOH | 70 | 97 |
| 8 | *^{t}*BuONa | *ⁱ*PrOH | >99 | 98 |
| 9 | *^{t}*BuOLi | *ⁱ*PrOH | 93 | 96 |

### Example 6 Preparation of Chiral Alcohol Intermediate Compound (3) (Ir-ligand Catalyst Investigation, S/C = 10000)

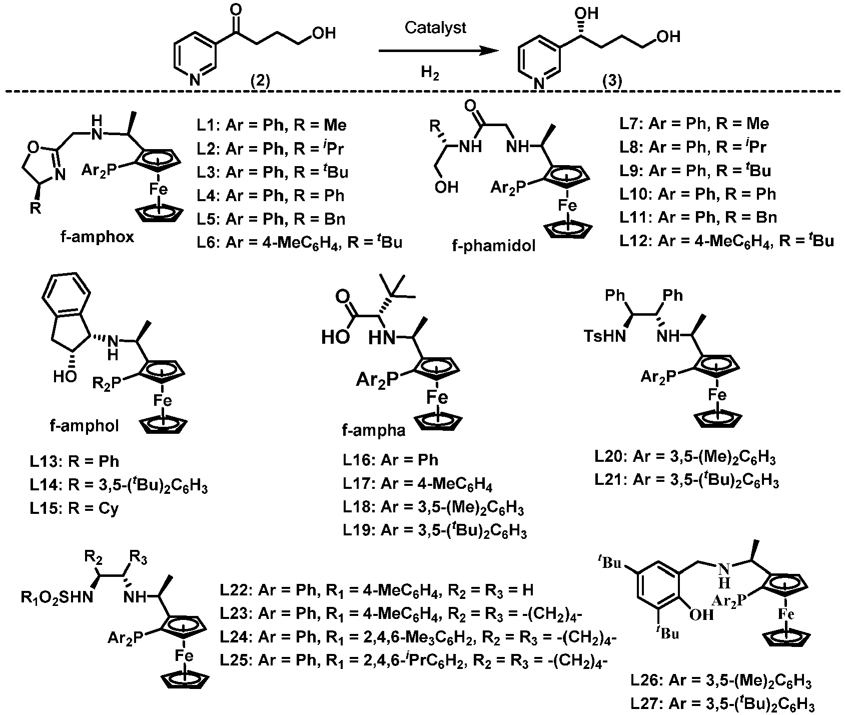

[Ir(COD)Cl]₂ (2.6 mg, 3.8 × 10⁻³ mmol) and a chiral ligand (8.4 × 10⁻³ mmol) were dissolved in 4 mL of isopropanol and stirred at room temperature for 3 h under argon atmosphere to give an orange clear catalyst solution. 200 µL of this orange solution was taken with a microsyringe and added to a mixed system of intermediate (2) (0.63 g, 3.82 mmol), isopropanol (4 mL) and potassium tert-butoxide (4.3 mg, 0.038 mmol). The reaction system was placed in an autoclave. The gas in the autoclave was purged with hydrogen three times, and finally 50 atm of hydrogen was introduced, and the mixture was reacted at 60 °C for 24 h. After the reaction was completed, the gas in the autoclave was slowly released, and the mixture was concentrated under reduced pressure to give 0.63 g of a yellow oily liquid, which was hydrogenation product (3). The conversion rate and ee value were determined by HPLC. The results are shown in Table 6 below.

**Table 6**

| No. | Ligand | S/C | Conversion rate (%) | ee (%) |
|---|---|---|---|---|
| 1 | **L3** | 10000 | >99 | 99 |
| 2 | **L7** | 10000 | >99 | 98 |
| 3 | **L8** | 10000 | >99 | 98 |
| 4 | **L9** | 10000 | >99 | >99 |
| 5 | **L10** | 10000 | >99 | 96 |
| 6 | **L12** | 10000 | 85 | 95 |
| 7 | **L14** | 10000 | >99 | 98 |
| 8 | **L16** | 10000 | 98 | 97 |
| 9 | **L19** | 10000 | 95 | 92 |
| 10 | **L20** | 10000 | >99 | 86 |
| 11 | **L21** | 10000 | >99 | 95 |
| 12 | **L22** | 10000 | >99 | 91 |
| 13 | **L24** | 10000 | >99 | 93 |
| 14 | **L25** | 10000 | >99 | 97 |
| 15 | **L27** | 10000 | 98 | 96 |

Example 7 To investigate the effect of the solvent in the reaction system on the reaction, on the basis of Example 6, L9 was used as the catalyst, potassium *tert*-butoxide was used as the base, and the solvent was sequentially replaced by MeOH, EtOH, EtOAc, DCM, THF, hexane, toluene, etc. The reaction time was 2 h, S/C = 10000. The results of the effect of different solvents on the conversion rate and ee value of the reduction of compound (2) are shown in Table 7 below. The conversion rate (conv.) and the enantioselectivity (ee) were determined by HPLC.

**Table 7**

| No. | Catalyst (n mol%) | Reaction solvent | Conversion rate (%) | ee (%) |
|---|---|---|---|---|
| 1 | **f-phamidol-L9** (0.01 mol %) | *ⁱ*PrOH | >99 | >99 |
| 2 | **f-phamidol-L9** (0.01 mol %) | MeOH | NR | - |
| 3 | **f-phamidol-L9** (0.01 mol %) | EtOH | 67 | 98 |
| 4 | **f-phamidol-L9** (0.01 mol %) | EtOAc | 20 | - |
| 5 | **f-phamidol-L9** (0.01 mol %) | DCM | 97 | 99 |
| 6 | **f-phamidol-L9** (0.01 mol %) | THF | 98 | >99 |
| 7 | **f-phamidol-L9** (0.01 mol %) | Hexane | 99 | >99 |
| 8 | **f-phamidol-L9** (0.01 mol %) | Toluene | 99 | >99 |

Example 8 To investigate the effect of the base added to the reaction system on the reaction, on the basis of Example 7, L9 was used as the catalyst, isopropanol was used as the solvent, and potassium *tert*-butoxide was sequentially replaced by potassium carbonate, cesium carbonate, potassium hydroxide, sodium hydroxide, sodium methoxide, potassium methoxide, sodium tert-butoxide, and lithium *tert*-butoxide. The reaction time was 12 h, S/C = 10000. The hydrogenation experiment was performed. The results are shown in Table 8 below.

**Table 8**

| No. | Base | Reaction solvent | Conversion rate (%) | ee (%) |
|---|---|---|---|---|
| 1 | *^{t}*BuOK | *ⁱ*PrOH | >99 | 99 |
| 2 | K₂CO₃ | *ⁱ*PrOH | >99 | 99 |
| 3 | Cs₂CO₃ | *ⁱ*PrOH | 90 | 95 |
| 4 | KOH | *ⁱ*PrOH | >99 | 99 |
| 5 | NaOH | *ⁱ*PrOH | >99 | 98 |
| 6 | NaOMe | *ⁱ*PrOH | >99 | 98 |
| 7 | KOMe | *ⁱ*PrOH | >99 | 99 |
| 8 | *^{t}*BuONa | *ⁱ*PrOH | >99 | 99 |
| 9 | *^{t}*BuOLi | *ⁱ*PrOH | >99 | 97 |

Example 9 Further, the catalyst f-phamidol-L9 was used as the catalyst, and isopropanol, a green solvent, was used as the solvent. The amount of the catalyst, the reaction time, and the like were separately changed. The results are shown in Table 9 below.

**Table 9**

| No. | S/C | Reaction temperature (°C) | Reaction time | Conversion rate (%) | ee (%) |
|---|---|---|---|---|---|
| 1 | 10000 | 25 | 12 h | >99 | >99 |
| 2 | 20000 | 25 | 12 h | >99 | >99 |
| 3 | 50000 | 25 | 24 h | >99 | >99 |
| 4 | 100000 | 25 | 24 h | >99 | >99 |
| 5 | 200000 | 25 | 48 h | 97 | 99 |
| 6 | 500000 | 25 | 72h | 36 | 99 |

### Example 10 Preparation of Chiral Alcohol Intermediate Compound (R)-(3) (Hectogram Scale, S/C = 100000)

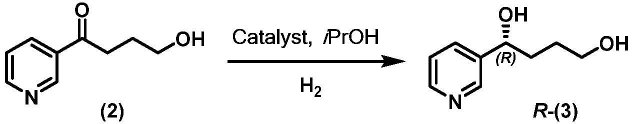

[Ir(COD)Cl]₂ (2.6 mg, 3.8 × 10⁻³ mmol) and the chiral ligand f-phamidol-L9 (4.8 mg, 8.4 × 10⁻³ mmol) were dissolved in 4 mL of isopropanol and stirred for well complexing at room temperature for 3 h under argon atmosphere to give an orange clear catalyst solution. Intermediate (2) (126 g, 0.763 mol), isopropanol (500 mL) and potassium tert-butoxide (853 mg, 7.6 mmol) were added to a hydrogenation kettle, and the catalyst solution described above was added to the reaction solution described above. The hydrogenation kettle was sealed. The gas in the autoclave was purged with hydrogen three times, and finally 50 atm of hydrogen was introduced, and the mixture was reacted at 25-30 °C for 48 h. After the reaction was completed, the gas in the autoclave was slowly released in a fume hood, and the mixture was concentrated under reduced pressure to give 126.9 g of a yellow oily liquid, which was hydrogenation product (*R*)-(3). The yield was quantified, and the *ee* value was determined 99% by chiral HPLC analysis. [α]²⁵_{D} = +45.8 (c = 1.0, CHCl₃); ¹H NMR (600 MHz, Chloroform-d) δ 8.42 (s, 1H), 8.34 - 8.28 (m, 1H), 7.69 (d, *J* = 7.8 Hz, 1H), 7.26 - 7.17 (m, 1H), 4.89 (br, 2H), 4.69 (t, *J* = 5.9 Hz, 1H), 3.60 (ddt, *J* = 18.4, 13.8, 7.0 Hz, 2H), 1.80 (q, *J* = 6.3 Hz, 2H), 1.62 (ddq, *J* = 26.8, 13.3, 6.6 Hz, 2H). ¹³C NMR (151 MHz, CDCl₃) δ 147.70, 147.08, 140.85, 134.08, 123.52, 71.24, 61.97, 36.23, 28.81.

### Example 11 Preparation of Chiral Alcohol Intermediate Compound (3) (Transfer Hydrogenation Catalyst Investigation)

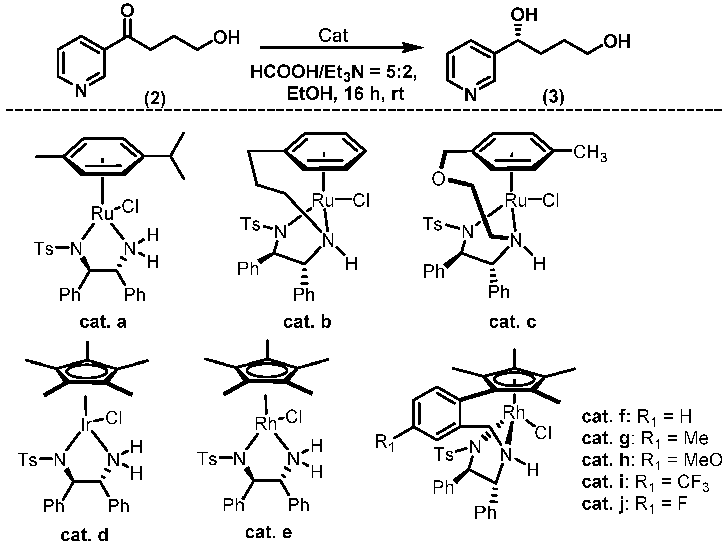

Compound 2 (0.2 mmol) was in ethanol (1 mL), and a mixture of the catalyst cat. (S/C = 1000) and 50 µL of formic acid-triethylamine (HCOOH/Et₃N, 5:2) (3.0 equivalents of [H], calculated by formic acid) were added at a temperature of 30 °C under argon or nitrogen atmosphere (the catalyst was 0.002 M ethanol solution, and 100 µL was used). Asymmetric transfer hydrogenation reaction was performed for 3 h tracked by TLC to give intermediate (3). The conversion rate and ee value were determined by HPLC. The results are shown in Table 10 below.

**Table 10**

| No. | Catalyst (n mol%) | Reaction time (h) | Conversion rate (%) | ee (%) |
|---|---|---|---|---|
| 1 | **cat. a** (0.1 mol %) | 24 | 36 | 34 |
| 2 | **cat. b** (0.1 mol %) | 24 | 60 | 60 |
| 3 | **cat. c** (0.1 mol %) | 24 | 80 | 80 |
| 4 | **cat. d** (0.1 mol %) | 24 | 62 | 78 |
| 5 | **cat. e** (0.1 mol %) | 24 | 46 | 43 |
| 6 | **cat. f** (0.1 mol %) | 6 | 99 | 91 |
| 7 | **cat. g** (0.1 mol %) | 6 | 99 | 92 |
| 8 | **cat. h** (0.1 mol %) | 6 | 99 | 93 |
| 9 | **cat. i** (0.1 mol %) | 6 | 99 | 92 |
| 10 | **cat. j** (0.1 mol %) | 6 | 99 | 90 |

Example 12 To further investigate the effect of the base added to the reaction system on the reaction, the following experiment was performed using **cat**. **h** as the catalyst (S/C = 1000) and ethanol as the solvent, and sequentially replacing triethylamine with diisopropylethylamine (DIPEA), diisopropylamine (*ⁱ*Pr_{2N}H), diethylamine (Et₂NH), DBU, and no base added on the basis of Example 11. The reaction time was 3 h, S/C =1000. The results are shown in Table 11 below.

**Table 11**

| No. | Hydrogen source (5:2) | Reaction solvent | Conversion rate (%) | ee (%) |
|---|---|---|---|---|
| 1 | HCOOH/Et₃N | EtOH | 99 | 93 |
| 2 | HCOOH/DIPEA | EtOH | 99 | 93 |
| 3 | HCOOH/*i*Pr₂NH | EtOH | 99 | 93 |
| 4 | HCOOH/Et₂NH | EtOH | 99 | 93 |
| 5 | HCOOH/DBU | EtOH | 99 | 94 |
| 6 | HCOOH/no base | EtOH | 70 | 93 |

Example 13 On the basis of Example 12, the hydrogen source formic acid-triethylamine (5:2) was further replaced with formic acid-sodium formate (3:1), formic acid-potassium formate (3:1), sodium formate, and potassium formate (3.0 equivalents of [H]). The reaction time was determined by TLC tracking, S/C = 1000. The reaction solvent was methanol, ethanol, or a mixed solution of methanol and water, and ethanol and water in a volume ratio of 1:1, and the following experiment was performed. The results of the effect on the conversion rate and ee value of the reduction of compound (2) are shown in Table 12 below. The results show that the reaction can be carried out even in aqueous solvents, and that the conversion rate and the enantioselectivity can both achieve very good results (> 90% conv., 98% ee).

**Table 12**

| No. | Hydrogen source | Reaction solvent | Conversion rate (%) | ee (%) |
|---|---|---|---|---|
| 1 | HCOOH/HCOONa (3:1) | EtOH | 98 | 93 |
| 2 | HCOOH/HCOOK (3:1) | EtOH | 99 | 93 |
| 3 | HCOONa | EtOH/H₂O | 93 | 93 |
| 4 | HCOOK | EtOH/H₂O | 91 | 93 |

Example 14 Further, the catalyst **cat. h** was used as the catalyst, and ethanol (EtOH), a green solvent, was used as the reaction solvent, and formic acid triethylamine (5:2) was used as the hydrogen source. The amount of the hydrogen source was 3.0 equivalents (calculated by formic acid). The reaction concentration was 0.5 M. The catalyst, the reaction time, the reaction temperature, and the like were separately changed. The results are shown in Table 13 below.

**Table 13**

| No. | S/C | Reaction temperature (°C) | Reaction time | Conversion rate (%) | ee (%) |
|---|---|---|---|---|---|
| 1 | 1000 | 30 | 6h | 99 | 93 |
| 2 | 2000 | 30 | 30 h | 99 | 93 |
| 3 | 5000 | 30 | 30 h | 77 | 93 |
| 4 | 5000 | 60 | 30h | >99 | 90 |

Example 15 On the basis of Example 11, the catalyst **cat. h** was used as the catalyst, and the solvent was sequentially replaced with MeOH, THF, EtOAc, DCM, MeCN, etc. The reaction was performed for 6 h. The results of the effect of different solvents on the conversion rate and ee value of the reduction of compound (2) are shown in Table 14 below.

**Table 14**

| No. | Hydrogen source | Reaction solvent | Conversion rate (%) | ee (%) |
|---|---|---|---|---|
| 1 | HCOOH/Et₃N(5:2) | EtOH | 99 | 93 |
| 2 | HCOOH/Et₃N(5:2) | MeOH | 99 | 93 |
| 3 | HCOOH/Et₃N(5:2) | THF | 95 | 92 |
| 4 | HCOOH/Et₃N(5:2) | EtOAc | 80 | 91 |
| 5 | HCOOH/Et₃N(5:2) | DCM | 89 | 93 |
| 6 | HCOOH/Et₃N(5:2) | MeCN | 75 | 92 |

### Example 16 Synthesis of (S)-Nicotine (Leaving Group LG Was OMs, Methylamine Alcohol Solution)

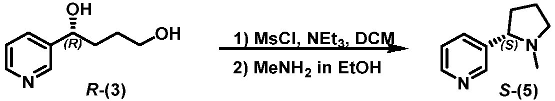

33.4 g (0.2 mol) of chiral alcohol intermediate (R)-(3) was weighed out. 200 mL of dichloromethane and 83.4 mL of triethylamine (0.6 mol) were added. The reaction system was placed in a -10 °C low-temperature cold bath. 38.8 mL of methanesulfonyl chloride (0.5 mol) was dissolved in 100 mL of dichloromethane and slowly added dropwise to the reaction system. After the dropwise addition was completed, the mixture was reacted at - 10 °C for 1 h. After the reaction was completed, the reaction was quenched with 100 mL of water. The aqueous phase was extracted three times with dichloromethane (100 mL × 3), and the organic phases were combined, washed once with 20 mL of saturated sodium bicarbonate solution, dried over anhydrous sodium sulfate, filtered, and concentrated to one fourth for later use. In a -10 °C low-temperature cold bath, 66 mL of methylamine alcohol solution (33 wt% in EtOH) was added dropwise to the concentrated solution obtained above, and the mixture was stirred at -10 °C for 24 h. After the reaction was completed, excess methylamine was directly removed by rotary evaporation, and the residue was diluted with water and extracted 3 times with DCM. The organic phase was dried and concentrated to give 30.8 g of (*S*)-nicotine crude product. The ee value was determined 99% by HPLC, and the purity was 97% by GC analysis. Compound (5) was obtained after distillation, namely 26.6 g of (*S*)-nicotine pure product, 82% yield. The ee value was determined 99% by HPLC, and the purity was 99.7% by GC analysis.

[α]²⁵_{D} = -98.5 (c = 1.0, CHCl₃), ¹H NMR (400 MHz, Chloroform-d) δ 8.59 (d, *J* = 1.8 Hz, 1H), 8.54 (dd, *J* = 4.8, 1.6 Hz, 1H), 7.76 (dt, *J* = 7.8, 1.8 Hz, 1H), 7.31 (dd, *J* = 7.8, 4.8 Hz, 1H), 3.33 - 3.25 (m, 1H), 3.14 (t, *J* = 8.3 Hz, 1H), 2.36 (q, *J* = 9.2 Hz, 1H), 2.26 (ddt, *J* = 12.7, 5.1, 2.1 Hz, 1H), 2.21 (s, 3H), 2.09 - 1.96 (m, 1H), 1.92 - 1.83 (m, 1H), 1.78 (dddd, *J* = 12.4, 11.1, 8.9, 5.6 Hz, 1H), ¹³C NMR (101 MHz, CDCl₃) δ 149.38, 148.47, 138.59, 134.78, 123.49, 68.74, 56.87, 40.24, 35.06, 22.47.

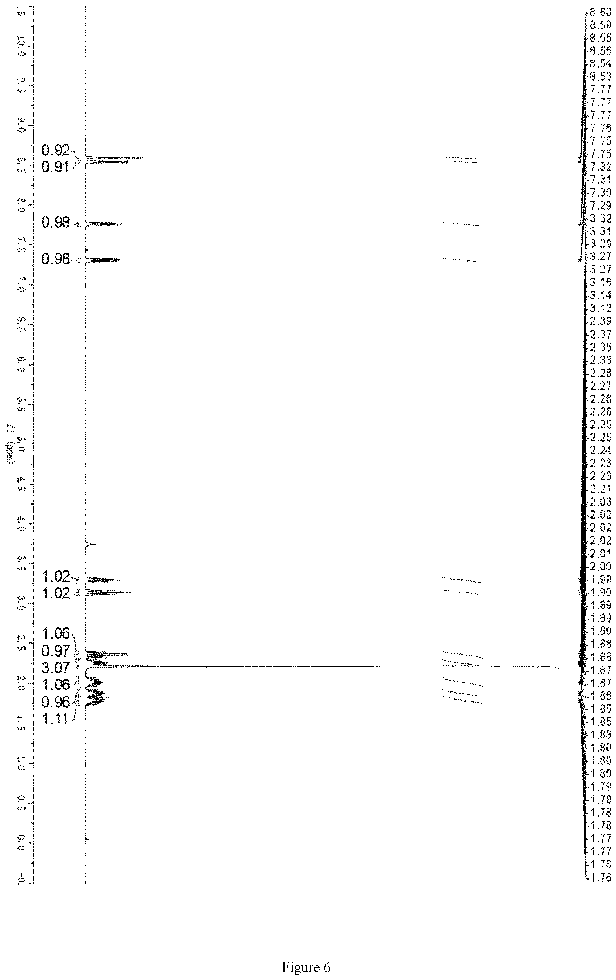

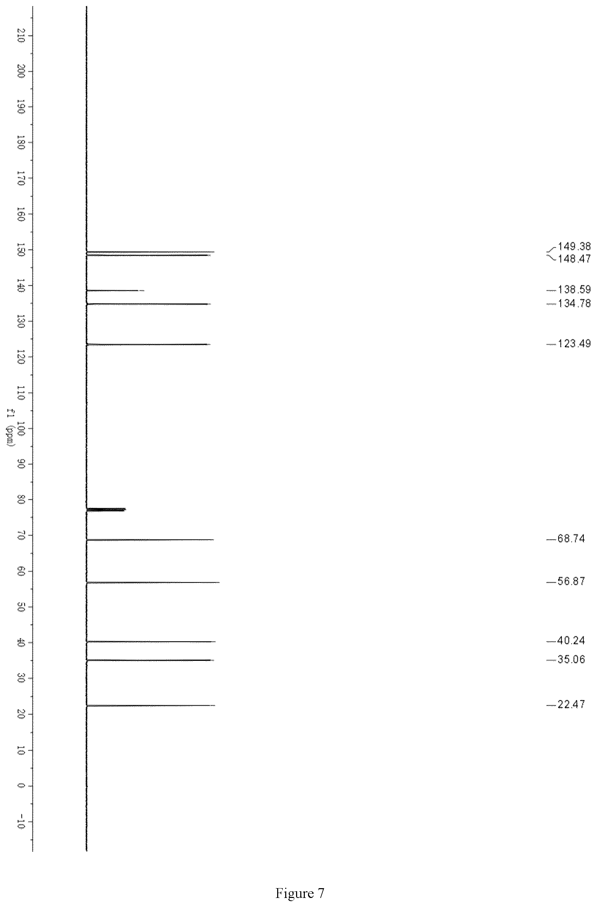

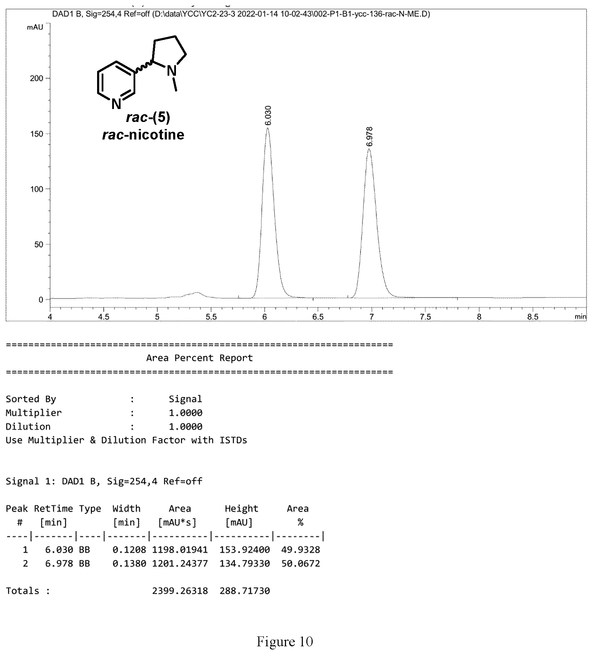

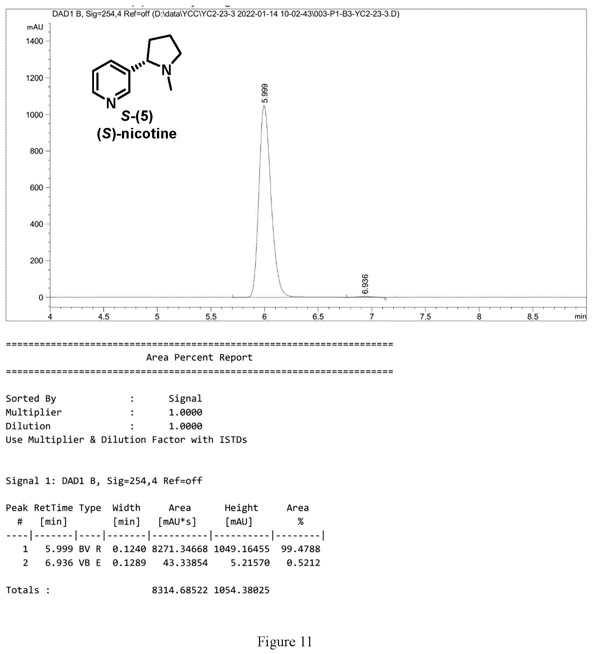

FIG. 6 is a ¹H NMR spectrum of compound (5), and FIG. 7 is a ¹³C NMR spectrum of compound (5). FIG. 10 is an HPLC chromatogram of racemic compound (5), and FIG. 11 is an HPLC chromatogram of chiral compound (5).

### Example 17 Synthesis of (S)-Nicotine (Leaving Group LG Was OTs, Methylamine Alcohol Solution)

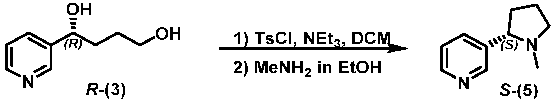

33.4 g (0.2 mol) of chiral alcohol intermediate (*R*)-(3) was weighed out and dissolved with 200 mL of dichloromethane, and 83.4 mL of triethylamine (0.6 mol) was added dropwise. The reaction system was placed in a -10 °C low-temperature cold bath. 95.3 g of *p*-toluenesulfonyl chloride (0.5 mol) was dissolved in 100 mL of dichloromethane and slowly added dropwise to the reaction system. After the dropwise addition was completed, the mixture was reacted at -10 °C for 3 h. After the reaction was completed, the reaction was quenched with 100 mL of water. The aqueous phase was extracted three times with dichloromethane (100 mL × 3), and the organic phases were combined, washed once with 20 mL of saturated sodium bicarbonate solution, dried over anhydrous sodium sulfate, filtered, and concentrated to one fourth for later use. In a -10 °C low-temperature cold bath, 66 mL of methylamine alcohol solution (33 wt% in EtOH) was added dropwise to the concentrated solution obtained above, and the mixture was stirred at -10 °C for 24 h. After the reaction was completed, excess methylamine was directly removed by rotary evaporation, and the residue was diluted with water and extracted 3 times with DCM. The organic phase was dried and concentrated to give 31.2 g of (*S*)-nicotine crude product. The ee value was determined 99% by HPLC, and the purity was 94% by GC analysis. 26.0 g of (*S*)-nicotine pure product was obtained after distillation, 82% yield. The ee value was determined 99% by HPLC, and the purity was 99.6% by GC analysis.

### Example 18 Synthesis of (S)-Nicotine (Leaving Group LG Was OMs, Methylamine Aqueous Solution)

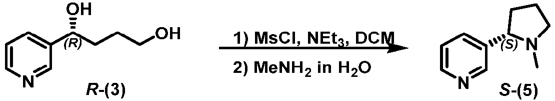

33.4 g (0.2 mol) of chiral alcohol intermediate (R)-(3) was weighed out. 200 mL of dichloromethane and 83.4 mL of triethylamine (0.6 mol) were added. The reaction system was placed in a -10 °C low-temperature cold bath. 38.8 mL of methanesulfonyl chloride (0.5 mol) was dissolved in 100 mL of dichloromethane and slowly added dropwise to the reaction system. After the dropwise addition was completed, the mixture was reacted at - 10 °C for 1 h. After the reaction was completed, the reaction was quenched with 100 mL of water. The aqueous phase was extracted three times with dichloromethane (100 mL × 3), and the organic phases were combined, washed once with 20 mL of saturated sodium bicarbonate solution, dried over anhydrous sodium sulfate, filtered, and concentrated to one fourth for later use. In a -10 °C low-temperature cold bath, 40 mL of methylamine alcohol solution (40 wt% in H₂O) was added dropwise to the concentrated solution obtained above, and the mixture was stirred at -10 °C for 24 h. After the reaction was completed, excess methylamine was directly removed by rotary evaporation, and the residue was diluted with water and extracted 3 times with DCM. The organic phase was dried and concentrated to give 31.6 g of (*S*)-nicotine crude product. The ee value was determined 99% by HPLC, and the purity was 95% by GC analysis. 26.9 g of (*S*)-nicotine pure product was obtained after distillation, 83% yield. The ee value was determined 99% by HPLC, and the purity was 99.6% by GC analysis.

### Example 19 Preparation of Chiral Alcohol Intermediate Compound (S)-(3) (S/C = 100000)

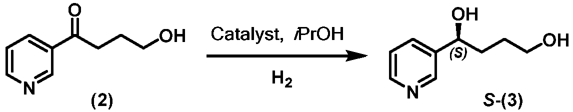

[Ir(COD)Cl]₂ (2.6 mg, 3.8 × 10⁻³ mmol) and the chiral ligand ent-f-phamidol-L9 (4.8 mg, 8.4 × 10⁻³ mmol) with the opposite configuration to Example 10 were dissolved in 4 mL of isopropanol and stirred for well complexing at room temperature for 3 h under argon atmosphere to give an orange clear catalyst solution. Intermediate (2) (63 g, 0.382 mol), isopropanol (250 mL), and potassium tert-butoxide (427 mg, 3.8 mmol) were added to a hydrogenation kettle, and 2 mL of the catalyst solution described above was added to the reaction solution described above. The hydrogenation kettle was sealed. The gas in the autoclave was purged with hydrogen three times, and finally 50 atm of hydrogen was introduced, and the mixture was reacted at 25-30 °C for 48 h. After the reaction was completed, the gas in the autoclave was slowly released in a fume hood, and the mixture was concentrated under reduced pressure to give 63.8 g of a yellow oily liquid, which was hydrogenation product (*S*)-(3). The yield was quantified, and the *ee* value was determined 99% by chiral HPLC analysis. The configuration of the product was the opposite to that of Example 10. The NMR characterization data of (*S*)-(3) was consistent with that of (*R*)-(3), and the optical rotation value was [α]²⁵_{D} = -45.0 (c = 1.0, CHCl₃).

### Example 20 Synthesis of (R)-Nicotine (Leaving Group LG Was OMs, Methylamine Alcohol Solution)

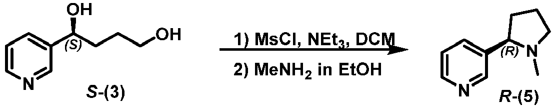

16.7 g of chiral alcohol intermediate (S)-(3) (0.1 mol) was weighed out. 100 mL of dichloromethane and 41.7 mL of triethylamine (0.3 mol) were added. The reaction system was placed in a -10 °C low-temperature cold bath. 19.4 mL of methanesulfonyl chloride (0.25 mol) was dissolved in 50 mL of dichloromethane and slowly added dropwise to the reaction system. After the dropwise addition was completed, the mixture was reacted at - 10 °C for 1 h. After the reaction was completed, the reaction was quenched with 50 mL of water. The aqueous phase was extracted three times with dichloromethane (50 mL × 3), and the organic phases were combined, washed once with 10 mL of saturated sodium bicarbonate solution, dried over anhydrous sodium sulfate, filtered, and concentrated to one fourth for later use. In a -10 °C low-temperature cold bath, 15 mL of methylamine alcohol solution (33 wt% in EtOH) was added dropwise to the concentrated solution obtained above, and the mixture was stirred at -10 °C for 24 h. After the reaction was completed, excess methylamine was directly removed by rotary evaporation, and the residue was diluted with water and extracted 3 times with DCM. The organic phase was dried and concentrated to give 15.8 g of (*R*)-nicotine crude product. The ee value was determined 99% by HPLC, and the purity was 94% by GC analysis. 13.0 g of (*R*)-nicotine pure product was obtained after distillation, 80% yield. The ee value was determined 99% by HPLC, and the purity was 99.7% by GC analysis.

The examples described above are preferred embodiments of the present disclosure, which, however, are not intended to limit the embodiments of the present disclosure. Any other changes, modifications, substitutions, combinations, and simplifications can be made without departing from the spirit and principle of the present disclosure, and should be construed as equivalent replacements and included in the protection scope of the present disclosure.

## Claims

1. A method for preparing a compound of formula (3), 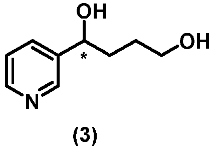
the compound having an *R* or *S* configuration at the stereoisomer center labeled with *;
an enantiomer excess of at least 70% relative to the opposite enantiomer,
wherein the method comprises the following steps: asymmetrically reducing the intermediate compound represented by formula (2): 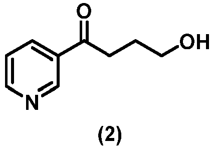
the asymmetric reduction is carried out in a suitable organic solvent in the presence of a chiral metal catalyst, a chiral ligand, a transition metal, an additive, and a hydrogen source, wherein the hydrogen source is selected from at least one of hydrogen, formic acid, a mixture of formic acid and formate, and a mixture of formic acid and organic amine, and the transition metal is selected from at least one of ruthenium, rhodium, iridium, palladium, manganese, copper, and iron.

2. The method for preparing the compound of formula (3) according to claim 1, wherein the chiral catalyst has the structure of formula I: 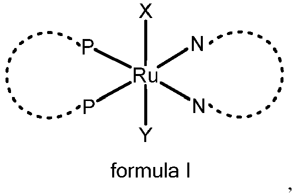 wherein X and Y are each independently halogen, acetate, or hydrogen; 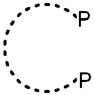 represents a diphosphine ligand; 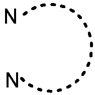 represents a diamine structure.

3. The method according to claim 2, wherein the diamine structure is selected from any one of the following structures or corresponding isomers thereof:

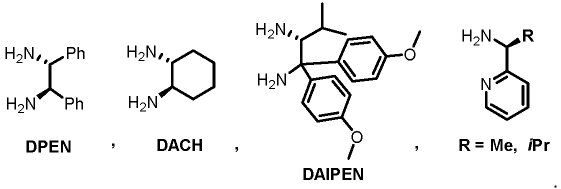

4. The method according to claim 2, wherein the diphosphine ligand is selected from at least one of Binap, H8-Binap, MeO-Biphep, C3*-Tunephos, Segphos, Synphos, SunPhos, Difluophos, P-Phos, BPE, DIPAMP, DIOP, Duphos, SDP, and O-SDP, and corresponding isomers thereof or derivatives thereof.

5. The method according to claim 4, wherein the diphosphine ligand is:
at least one of

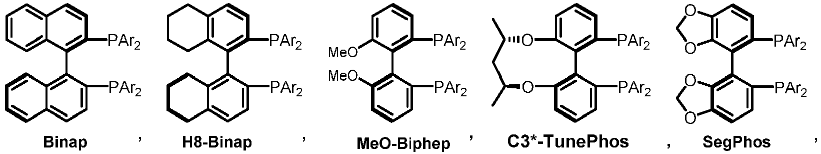

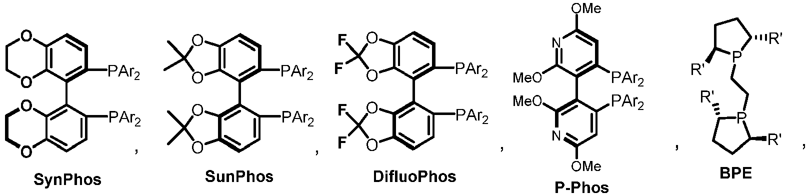

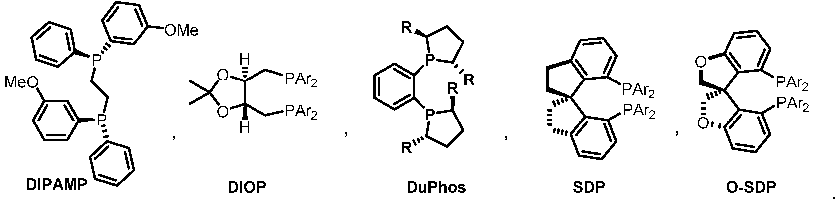

6. The method according to claim 1 or 2, wherein the chiral catalyst is selected from: 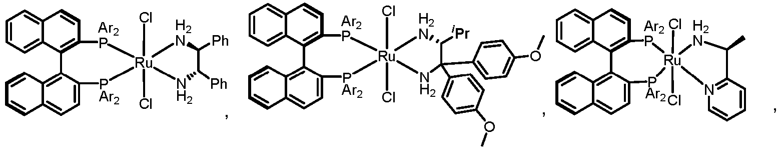 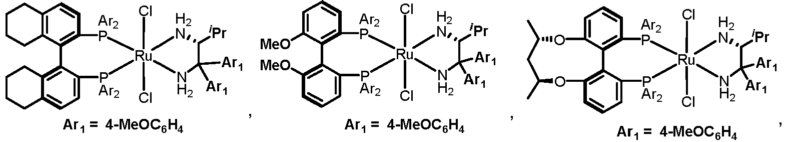 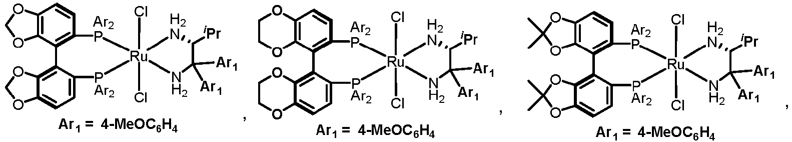 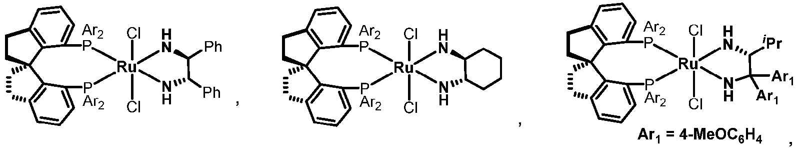 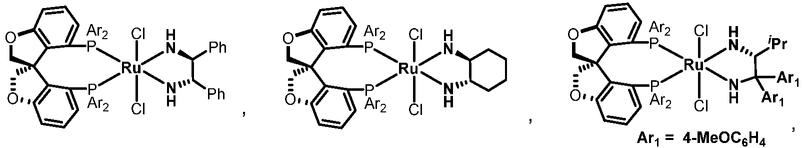 wherein the Ar group in the diphosphine ligand represents aryl and is selected from at least one of phenyl, 4-methylphenyl, 3,5-dimethylphenyl, 3,5-di-tert-butylphenyl, or 3,5-di-tert-butyl 4-methoxyphenyl.

7. The method according to claim 1, wherein the chiral catalyst is obtained by complexing a metal precursor and a chiral ligand, wherein the metal is selected from ruthenium, rhodium, iridium, or palladium, and the chiral ligand is selected from at least one of L1-L27:

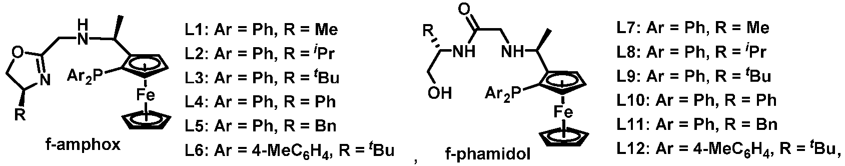

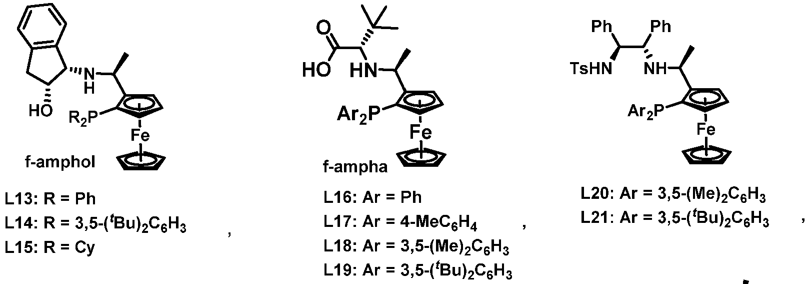

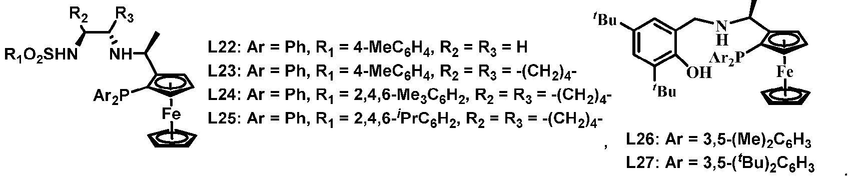

8. The method according to any one of claims 1-6, wherein the hydrogen source is hydrogen; and/or the organic solvent is selected from at least one of methanol, ethanol, isopropanol, tetrahydrofuran, dichloromethane, and toluene; and/or the additive is a base, and is selected from at least one of potassium *tert*-butoxide, sodium *tert-*butoxide, lithium *tert*-butoxide, potassium hydroxide, sodium hydroxide, sodium carbonate, potassium carbonate, and cesium carbonate.

9. The method according to claim 1, wherein the chiral catalyst is selected from: 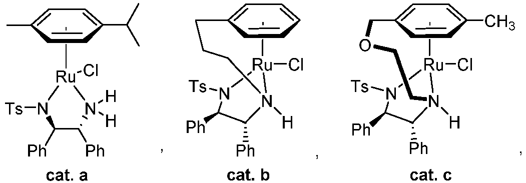 at least one of

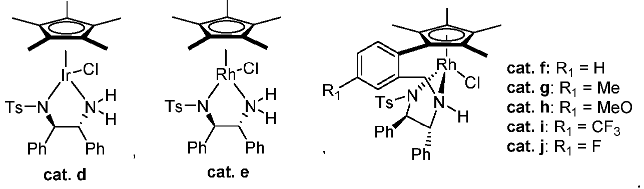

10. The method according to claim 1 or 9, wherein the organic solvent is selected from at least one of EtOAc, CH₂Cl₂, ClCH₂CH₂Cl, MeOH, EtOH, iPrOH, (HOCH₂)₂, THF, and PhMe; and/or the hydrogen source is selected from at least one of HCOOH/Et₃N, HCOOH/DIPEA, HCOOH/*i*Pr₂NH, HCOOH/Et₂NH, HCOOH/DBU, HCOOH/HCOOK, and HCOOH/HCOONa.

11. A compound, having the structure of the following formula (3): 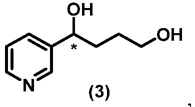 and specifically comprising two configurations (*R*) and (*S*), wherein the structures thereof are shown below, 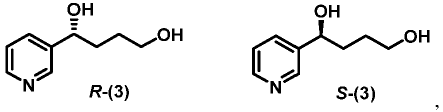 wherein *R*-(3) and *S*-(3) can be prepared by any one of the methods according to claims 1-10.

12. An asymmetric process for preparing nicotine, wherein the synthetic route is as follows: 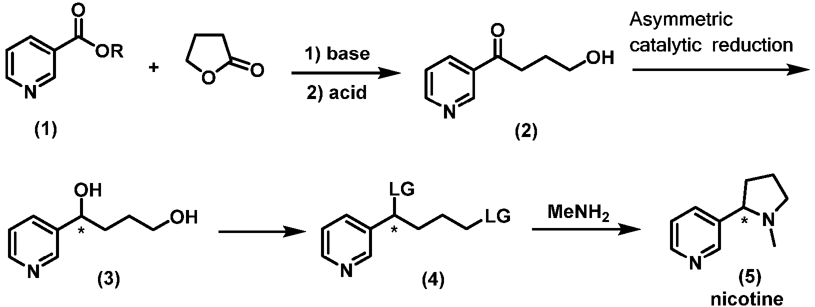 wherein R in compound (1) represents alkyl, and the leaving group LG in compound (4) represents halogen or sulfonate, wherein the sulfonate is selected from at least one of methanesulfonate, trifluoromethanesulfonate, *p*-toluenesulfonate, and nitrosulfonate, and intermediate (3) is prepared by the synthesis method according to any one of claims 1-11.
